(19) Europäisches Patentamt
European Patent Office
Office européen des brevets

(11) EP 1 028 947 B1

(12) **EUROPEAN PATENT SPECIFICATION**

(45) Date of publication and mention of the grant of the patent:
**12.06.2002 Bulletin 2002/24**

(21) Application number: **98956442.2**

(22) Date of filing: **05.11.1998**

(51) Int Cl.[7]: **C07D 233/64**, A61K 31/415

(86) International application number:
**PCT/US98/23223**

(87) International publication number:
**WO 99/24405 (20.05.1999 Gazette 1999/20)**

(54) **H3 RECEPTOR LIGANDS OF THE PHENYL-ALKYL-IMIDAZOLES TYPE**

PHENYLALKYL IMIDAZOLE DERIVATE ALS H3 REZEPTOR LIGANDEN

LIGANDS DU RECEPTEUR H3 DU TYPE PHENYL-ALKYL-IMIDAZOLE

(84) Designated Contracting States:
**AT BE CH DE DK ES FI FR GB GR IE IT LI LU NL PT SE**
Designated Extension States:
**LT LV RO**

(30) Priority: **07.11.1997 US 966344**
**05.08.1998 US 129711**

(43) Date of publication of application:
**23.08.2000 Bulletin 2000/34**

(73) Proprietor: **SCHERING CORPORATION**
**Kenilworth, New Jersey 07033-0530 (US)**

(72) Inventors:
- **ASLANIAN, Robert, G.**
**Rockaway, NJ 07866 (US)**
- **McCORMICK, Kevin, D.**
**Edison, NJ 08820 (US)**
- **MUTAHI, Mwangi, Wa**
**Edison, NJ 08837 (US)**

(74) Representative: **Ritter, Stephen David et al**
**Mathys & Squire**
**100 Gray's Inn Road**
**London WC1X 8AL (GB)**

(56) References cited:
**WO-A-93/14070** **WO-A-95/14007**
**WO-A-96/29315** **WO-A-98/06394**

Remarks:
The file contains technical information submitted after the application was filed and not included in this specification

EP 1 028 947 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

## Description

FIELD OF THE INVENTION

The present invention relates to a phenyl-alkyl-imidazole having valuable pharmacological properties especially CNS activities and activity against inflammatory disease. The Compound of this invention is an antagonist of the $H_3$ receptor.

BACKGROUND OF THE INVENTION

European Patent Application No. 0 420 396 A2 (Smith Kline & French Laboratories Limited) and Howson *et al*., *Bioorg. & Med. Chem. Letters,* Vol. 2 No. 1 (1992), pp. 77-78 describe imidazole derivatives having an amidine group as $H_3$ agonists. Van der Groot et al. (*Eur. J. Med. Chem.* (1992) Vol. 27, pp. 511-517) describe isothiourea analogs of histamine as potent agonists or antagonists of the histamine $H_3$ receptor, and these isothiourea analogs of histamine overlap in part with those of the two references cited above. Clapham et al. ["Ability of Histamine $H_3$ Receptor Antagonists to improve Cognition and to increase Acetylcholine Release *in vivo* in the Rat", British Assn. for Psychopharmacology, July 25-28 1993, reported in *J. Psychopharmacol.* (Abstr. Book), A17] describe the ability of histamine $H_3$ receptor antagonists to improve cognition and to increase release of acetylcholine in *vivo* in the rat. Clapham et al. ["Ability of the selective Histamine $H_3$ Receptor Antagonist Thioperamide to improve Short-term Memory and Reversal Learning in the Rat", *Brit. J. Pharm. Suppl*., *1993*, 110, Abstract 65P] present results showing that thioperamide can improve short-term memory and reversal learning in the rat and implicate the involvement of $H_3$ receptors in the modulation of cognitive function. Yokoyama et al. ["Effect of thioperamide, a histamine $H_3$ receptor antagonist, on electrically induced convulsions in mice", *Eur. J. Pharmacol*., vol. 234 (1993), pp. 129-133] report how thioperamide decreased the duration of each phase of convulsion and raised the electroconvulsive threshold, and go on to suggest that these and other findings support the hypothesis that the central histaminergic system is involved in the inhibition of seizures. International Patent Publication No. WO9301812-A1 (SmithKline Beecham PLC) describes the use of S-[3-(4(5)-imidazolyl)propyl]isothiourea as a histamine $H_3$ antagonist, especially for treating cognitive disorders, *e.g.* Alzheimer's disease and age-related memory impairment. Schlicker et al. ["Novel histamine $H_3$ receptor antagonists: affinities in an $H_3$ receptor binding assay and potencies in two functional $H_3$ receptor models"] describe a number of imidazolylalkyl compounds wherein the imidazolylalkyl group is bonded to a guanidine group, an ester group or an amide group (including thioamide and urea), and compare these to thioperamide. Leurs et al. ["The histamine $H_3$-receptor: A target for developing new drugs", *Progr. Drug Res.* (1992) vol. 39, pp. 127-165] and Lipp et al. ["Pharmacochemistry of $H_3$-receptors" in *The Histamine Receptor,* eds.: Schwartz and Haas, Wiley-Liss, New York (1992), pp. 57-72] review a variety of synthetic $H_3$ receptor antagonists, and Lipp et al. (*ibid.*) have defined the necessary structural requirements for an $H_3$ receptor antagonist.

WO 95/14007 claims $H_3$ receptor antagonists of the formula

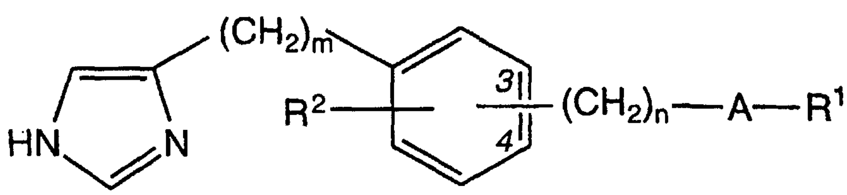

I

wherein

A is selected from -O-CO-NR$^1$-, -O-CO-, -NR$^1$-CO-NR$^1$-, -NR$^1$-CO-, -NR$^1$-, -O-, -CO-NR$^1$-, -CO-O-, and -C(:NR$^1$) -NR$^1$-; the groups $R^1$, which may be the same or different when there are two or three such groups in the molecule of formula I, are selected from hydrogen, and lower alkyl, aryl, cycloalkyl, heterocyclic and heterocycloalkyl groups, and groups of the formula $-(CH_2)_y$-G, where G is selected from $CO_2R^3$, $COR^3$, $CONR^3R^4$, $OR^3$, $SR^3$, $NR^3R^4$, heteroaryl and phenyl, which phenyl is optionally substituted by halogen, lower alkoxy or polyhaloloweralkyl, and y is an integer from 1 to 3;

$R^2$ is selected from hydrogen and halogen atoms, and alkyl, alkenyl, alkynyl and trifluoromethyl groups, and groups of the formula $OR^3$, $SR^3$ and $NR^3R^4$;

$R^3$ and $R^4$ are independently selected from hydrogen, and lower alkyl and cycloalkyl groups, or $R^3$ and $R^4$ together with the intervening nitrogen atom can form a saturated ring containing 4 to 6 carbon atoms that can be substituted with one or two lower alkyl groups;

with the proviso that, when y is 1 and G is $OR^3$, $SR^3$ or $NR^3R^4$, then neither $R^3$ nor $R^4$ is hydrogen;

the group $-(CH_2)_n$-A-$R^1$ is at the 3- or 4-position, and the group $R^2$ is at any free position;
m is an integer from 1 to 3; and
n is 0 or an integer from 1 to 3;
or a pharmaceutically acceptable acid addition salt thereof;
or a pharmaceutically acceptable salt thereof with a base when G is $CO_2H$; including a tautomeric form thereof.

The compounds are useful for treating various disorders, in particular such caused by allergy-induced responses. However, WO 95/14007 does not specifically disclose the compound of the present invention.

US Application Serial. No. 08/689951 filed August 16, 1996 and U.S. Application Serial No. 08/909319 filed August 14, 1997 disclose compositions for the treatment of the symptoms of allergic rhinitis using a combination of at least one histamine $H_1$ receptor antagonist and at least one histamine $H_3$ receptor antagonist.

In view of the art's interest in compounds which affect the $H_3$ receptors, novel compounds having antagonist activity on $H_3$ receptors would be a welcome contribution to the art. This invention provides just such a contribution by providing a novel compound having $H_3$ antagonist activity.

It has now been found that the compound:

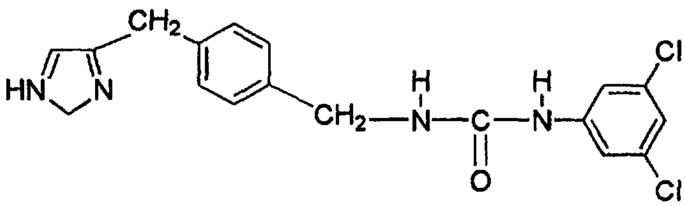

is particularly active and shows valuable pharmacological properties.

The invention further provides a pharmaceutical composition containing as an active ingredient, the above compound (or salt, solvate or tautomer thereof) together with a pharmaceutical carrier or excipient.

Further features of the invention are the use of the compound in the manufacture of a medicament for the treatment of allergy (for example asthma), inflammation, cardiovascular disease, hypertension, raised intraocular pressure (such as glaucoma)-i.e. a method of lowering intraocular pressure, sleeping disorders (e.g. hypersomnia, somnolence, narcolepsy and sleeplessness, such as insomnia), diseases of the GI tract, states of hyper and hypo motility and acidic secretion of the gastrointestinal tract, disturbances of the central nervous system, hypo and hyperactivity of the central nervous system (for example, agitation and depression) and other CNS disorders (such as Alzheimer's, schizophrenia, obesity and migraine).

Another feature of this invention is the use of the above compound or a salt, solvate or tautomer thereof in the manufacture of a medicament for treating inflammation.

Another feature of this invention is the use of the above compound or a salt, solvate or tautomer thereof in the manufacture of a medicament for treating allergy.

Another feature of this invention is the use of the above compound or a salt, solvate or tautomer thereof in the manufacture of a medicament for treating diseases of the GI-tract.

Another feature of this invention is the use of the above compound or a salt, solvate or tautomer thereof in the manufacture of a medicament for treating cardiovascular disease.

Another feature of this invention is the use of the above compound or a salt, solvate or tautomer thereof in the manufacture of a medicament for treating disturbances of the central nervous system.

The invention further provides a pharmaceutical composition comprising the above compound in combination with a histamine $H_1$ receptor antagonist.

The invention further provides the use of the above compound or salt, solvate or tautomer thereof, in combination or admixture with a histamine $H_1$ receptor antagonist in the manufacture of a medicament for the treatment of allergy-induced airway (e.g. upper airway) responses.

## DETAILED DESCRIPTION OF THE INVENTION

The compound of the invention is basic arid form pharmaceutically acceptable salts with organic and inorganic acids. Examples of suitable acids for such salt formation are hydrochloric, sulfuric, phosphoric, acetic, citric, oxalic, malonic, salicylic, malic, fumaric, succinic, ascorbic, maleic, methanesulfonic and other mineral and carboxylic acids well known to those skilled in the art. The salts are prepared by contacting the free base form with a sufficient amount of the desired acid to produce a salt in the conventional manner. The free base form may be regenerated by treating the salt with a suitable dilute aqueous base solution such as dilute aqueous sodium hydroxide, potassium carbonate,

ammonia and sodium bicarbonate. The free base form differs from their corresponding salt form somewhat in certain physical properties, such as solubility in polar solvents, but the salts are otherwise equivalent to the corresponding free base form for purposes of this invention.

The compound of the invention can exist in unsolvated as well as solvated form including hydrated forms, e. g., hemi-hydrate. In general, the solvated forms, with pharmaceutically acceptable solvents such as water, ethanol and the like are equivalent to the unsolvated form for purposes of the invention.

Numerous chemical substances are known to have histamine $H_1$ receptor antagonist activity. Many useful compounds can be classified as ethanolamines, ethylenediamines, alkylamines, phenothiazines or piperidines. Representative $H_1$ receptor antagonists include, without limitation: astemizole, azatadine, azelastine, acrivastine, brompheniramine, cetirizine, chlorpheniramine, clemastine, cyclizine, carebastine, cyproheptadine, carbinoxamine, descarboethoxyloratadine (also known as SCH-34117), diphenhydramine, doxylamine, dimethindene, ebastine, epinastine, efletirizine, fexofenadine, hydroxyzine, ketotifen, loratadine, levocabastine, mizolastine, mequitazine, mianserin, noberastine, meclizine, norastemizole, picumast, pyrilamine, promethazine, terfenadine, tripelennamine, temelastine, trimeprazine and triprolidine. Other compounds can readily be evaluated to determine activity at $H_1$ receptors by known methods, including specific blockade of the contractile response to histamine of isolated guinea pig ileum. See for example, WO98/06394 published February 19, 1998.

For example, the $H_3$ antagonist of this invention can be combined with an $H_1$ antagonist selected from astemizole, azatadine, azelastine, brompheniramine, cetirizine, chlorpheniramine, clemastine, carebastine, descarboethoxyloratadine (also known as SCH-34117), diphenhydramine, doxylamine, ebastine, fexofenadine, loratadine, levocabastine, mizolastine, norastemizole, or terfenadine.

Also, for example, the $H_3$ antagonist of this invention can be combined with an $H_1$ antagonist selected from, azatadine, brompheniramine, cetirizine, chlorpheniramine, carebastine, descarboethoxyloratadine (also known as SCH-34117), diphenhydramine, ebastine, fexofenadine, loratadine, or norastemizole.

Representative combinations include: the $H_3$ antagonist of this invention with loratadine, the $H_3$ antagonist of this invention with descarboethoxyloratadine, the $H_3$ antagonist of this invention with fexofenadine, and the $H_3$ antagonist of this invention with cetirizine.

Those skilled in the art will know that the term "upper airway" means the upper respiratory system--i.e., the nose, throat, and associated structures.

PROCESSES FOR PREPARING THE COMPOUND

The compound of the invention can be prepared by a process in which the left-hand part of the molecule represented by

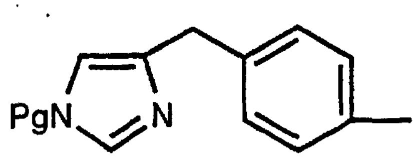

25

is coupled to a compound providing the remainder of the molecule. Those skilled in the art will appreciate that Pg represents a suitable protecting group (e.g., trityl, abbreviated "Tr"),

For the preparation of a compound of the formula I wherein A is $-CH_2-NH-CO-NH-$, reaction of an amino compound with an isocyanate:

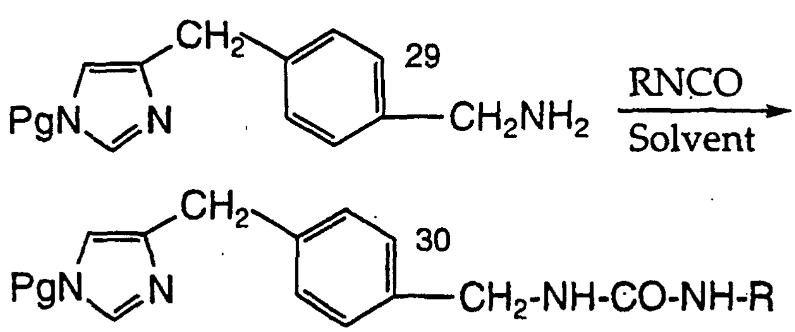


29

30

The amino compound can be prepared for example by reduction of the compound of the formula 28 wherein Y is CN (i.e. Compound 28B - shown below) with a hydride reducing agent such as lithium aluminum hydride, or by catalytic hydrogenation with *e.g.* Raney nickel or palladium on carbon.

PREPARATION OF STARTING MATERIALS AND INTERMEDIATES

Starting materials for the above processe can be prepared by the methods discussed below, wherein: Y represents a group convertible into -$CH_2$-$NH_2$. In a first step compound 28 is prepared:

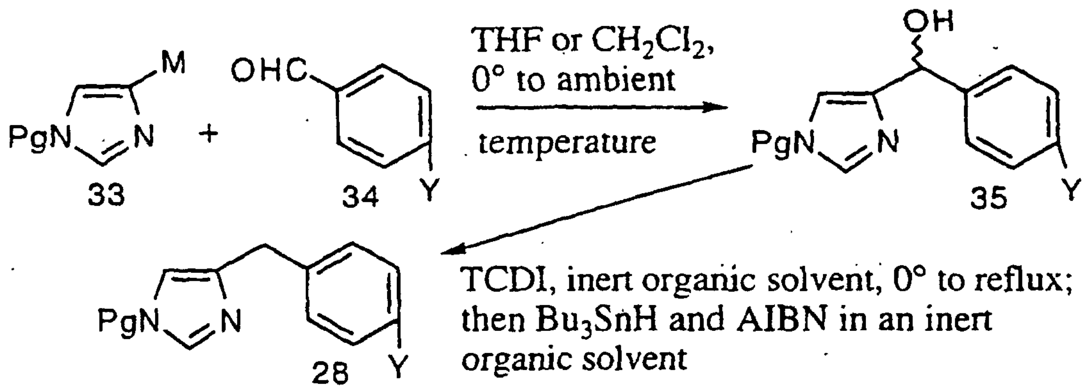


M stands for MgX (X= Br or I), Y stands for CN or TCDI stands for thiocarbonyldiimidazole, and AIBN stands for azoisobutylnitrile.

Reduction of the compound 28 wherein Y = CN, i.e., compound 28B, forms the corresponding amino compound 29:

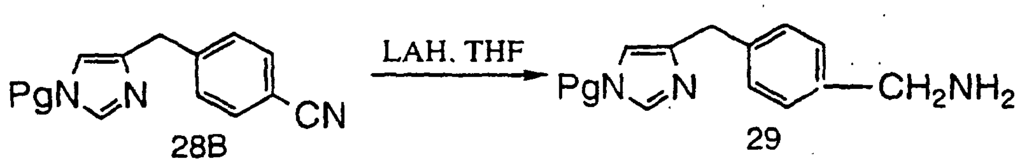


The last step is mostly the deprotection of any protecting groups. This can be accomplished by several methods well known in the art. The protecting group is preferably one that can be removed by hydrolysis or hydrogenolysis; it can for example be a trityl group $(C_6H_5)_3C$-, which is preferably removed by hydrolysis in an aqueous organic solvent. The hydrolysis can for example be effected by means of mineral acid in an aqueous water-miscible organic solvent such as a lower alkanol, especially methanol or ethanol. Other protecting groups that can be used (and their method of removal) include t-Bu-OCO- [often abbreviated to t-BOC] (which can be removed with acid, or with hydrazine, ammonia and a lower alkanol, e.g., methanol or ethanol), and (2-triloweralkylsilyl)-ethoxymethyl groups, especially $Me_3Si(CH_2)_2OCH_2$- [often abbreviated to SEM] (which can be removed with acid or fluoride ion).

Example 1

Step 1

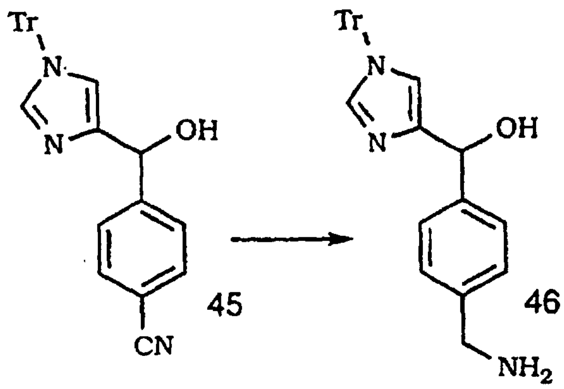

45 46

To a 1 L Parr flask containing 5.2 g of Raney nickel (washed with ethanol (4 x 10 mL)) was added 400 mL of $NH_3$ sat. methanol and 9.8 g of hydroxy nitrile compound 45 (22.2 mmol), and the mixture was hydrogenated at 45 psi $H_2$ for 8 h. The reaction mixture was filtered and concentrated. The product was purified by flash column (silica gel) eluting with 20:1:0.1 to 10:1:0.1 $CH_2Cl_2$-MeOH-$NH_3$aq. to give 7.65 g of desired product 46 (17.2 mmol, 77 % yield).

$^1$H -NMR ($CD_3OD$) 7.2-7.5 (20H, m), 6.84 (1H, s), 5.78 (1H, s), 5.78 (1H, s) and 3.82 (2H, s).

Step 2

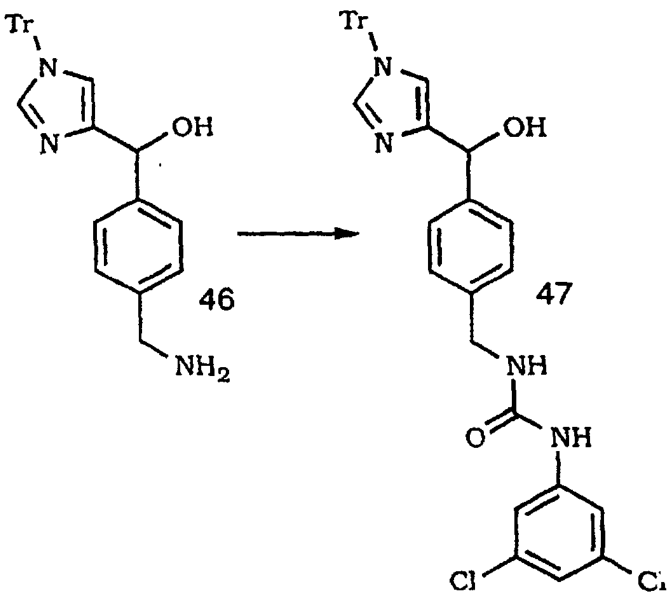

46 47

To a solution of compound 46 (7.65 g, 17.2 mmol) dissolved in THF (700 mL) was added a solution of 3,5-dichlorophenyl isocyanate (3.27 g, 17.4 mmol) dissolved in THF (50 mL). The reaction mixture was left stirring over night and concentrated to give clean crude product 47 as a white foam. The crude product was used in the next step without further purification.

$^1$H -NMR ($CD_3OD$ and $d_6$-DMSO) 7.2-7.5 (22H, m), 7.08 (1H, s), 6.84 (1H, s), 5.77 (1H, s), 4.43 (2H, s).

Step 3

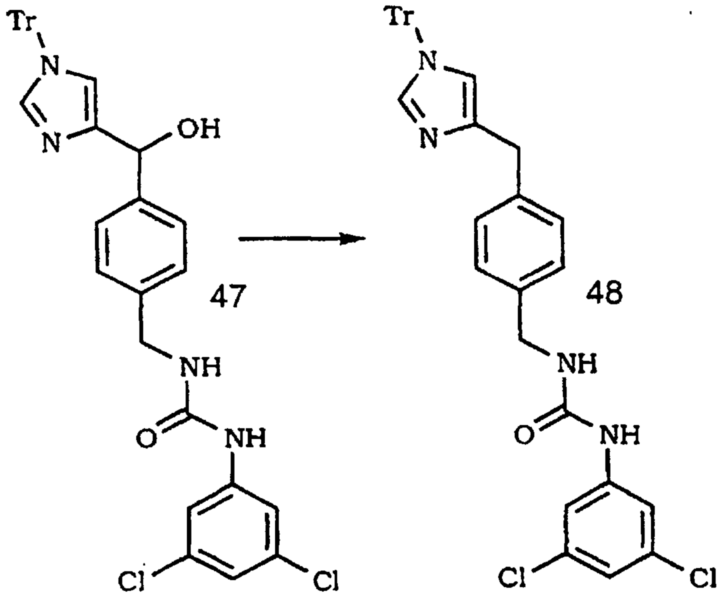

47

48

To a solution of crude compound 47 (~17.2 mmol) and NaI (21.5 g, 144 mmol) in $CH_2Cl_2$ (200 mL) and acetone (200 mL) was added 12.6 mL of dichloro dimethylsilane (103 mmol). After stirring at room temperature for 30 min, the reaction mixture was added to $CH_2Cl_2$ (500 mL) and washed with 10% sodium thiosulfate (500 mL, 4 x 250 mL), $H_2O$ (250 mL) and brine (250 mL), dried with $MgSO_4$ and concentrated. The product was purified by flash column (silica gel) eluting with 50:1 to 20:1 $CH_2Cl_2$-MeOH to give 9.10 g of desired product 48 as a white solid (14.7 mmol, 86% yield for two steps).

$^1$H -NMR ($CDCl_3$) 8.71 (1H, s), 7.44 (1H, s), 7.1-7.4 (H, m), 6.88 (1H, s), 6.71 (1H, s), 5.99 1H, d, J = 5.8 Hz), 4.15 (2H, d, J = 5.8 Hz), 3.80 (2H, s).

Step 4

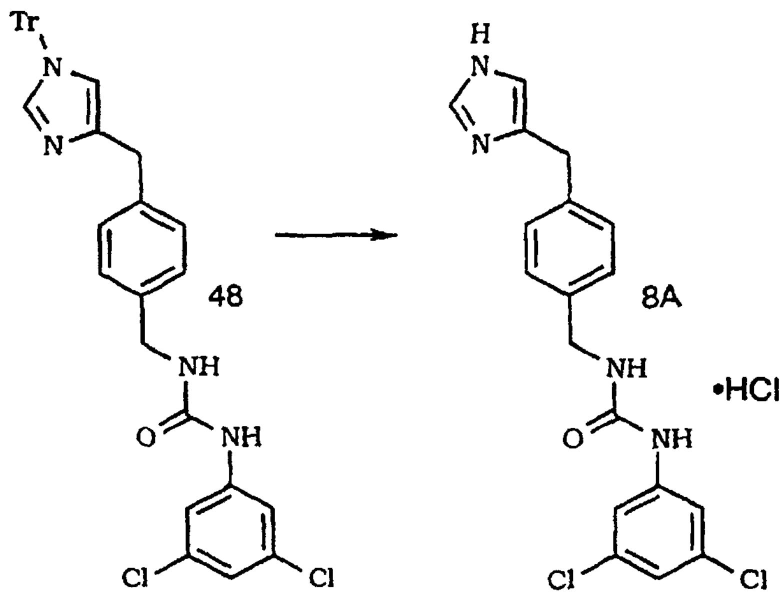

48

8A

•HCl

The trityl group was removed and converted to its HCl salt by standard procedures. The HCl salt was crystallized from EtOH - *tert*-butyl methyl ether to give desired product 8A white crystals (m.p. 182.5-184° C).

HRMS (FAB, M + H$^+$): mle calc'd for $[C_{18}H_{17}Cl_2N_4O]^+$: 375.0779, found 375.0787.

$^1H$ -NMR ($CD_3OD$) 8.88 (1H, s), 7.50 (2H, d, J = 1.8 Hz), 7.40 (2H, d, J = 8.1 Hz), 7.37 (1H, s), 7.32 (2H, d, J = 8.1 Hz), 7.05 (1H, t, J = 1.8 Hz), 4.44 (2H, s), 4.15 (2H, s).

It has surprisingly been found that the present compound is substantially more active than the preferred compounds of WO 95/14007. However, the most substantial advantage is that it provides higher blood levels of the compound and are believed to be more bioavailable and more easily absorbed orally. This make the compound of the present invention particularly useful as a medicament.

$H_3$-Receptor Binding Assay

The source of the $H_3$ receptors in this experiment was guinea pig brain. The animals weighed 400-600 g. The brain tissue was homogenized using a Polytron in a solution of 50 mM Tris, pH 7.5. The final concentration of tissue in the homogenization buffer was 10% w/v. The homogenates were centrifuged at 1,000 x g for 10 min. in order to remove clumps of tissue and debris. The resulting supematants were then centrifuged at 50,000 x g for 20 min. in order to sediment the membranes, which were next washed three times in homogenization buffer (50,000 x g for 20 min. each). The membranes were frozen and stored at -70°C until needed.

The compound to be tested was dissolved in DMSO and then diluted into the binding buffer (50 mM Tris, pH 7.5) such that the final concentration was 2 µg/ml with 0.1% DMSO. Membranes were then added (400 µg of protein) to the reaction tubes. The reaction was started by the addition of 3 nM [$^3$H]R-a-methylhistamine (8.8 Ci/mmol) or 3 nM [$^3$H]$N^a$-methylhistamine (80 Ci/mmol) and continued under incubation at 30°C for 30 min. Bound ligand was separated from unbound ligand by filtration, and the amount of radioactive ligand bound to the membranes was quantitated by liquid scintillation spectrometry. All incubations were performed in duplicate and the standard error was always less than 10%. The Compound was serially diluted to determine a $K_i$ (nM). The results are given in the table below for the HCl salt of the indicated compound.

The compound of the present invention had $K_i$ values of 4 and 12 nM.

From these test results and the background knowledge about the compounds described in the references in the section "Background of the Invention", it is to be expected that the compound of the invention would be useful in treating inflammation, allergy, diseases of the GI-tract, cardiovascular disease, or disturbances of the central nervous system.

Pharmaceutically acceptable inert carriers used for preparing pharmaceutical compositions from the compound of the invention and its salts can be either solid or liquid. Solid form preparations include powders, tablets, dispersible granules, capsules, cachets and suppositories. The powders and tablets may comprise from about 5 to about 70 percent active ingredient. Suitable solid carriers are known in the art, e.g. magnesium carbonate, magnesium stearate, talc, sugar, lactose. Tablets, powders, cachets and capsules can be used as solid dosage forms suitable for oral administration.

Liquid form preparations include solutions, suspensions and emulsions, for example water or water-propylene glycol solutions for parenteral injection. Liquid form preparations may also include solutions for intranasal administration.

Also included are solid form preparations which are intended for conversion, shortly before use, into liquid form preparations for either oral or parenteral administration. Such liquid forms include solutions, suspensions and emulsions.

Aerosol preparations suitable for inhalation may include solutions and solids in powder form, which may be in combination with a pharmaceutically acceptable carrier, such as an inert compressed gas.

For preparing suppositories, a low melting wax such as a mixture of fatty acid glycerides or cocoa butter is first melted, and the active ingredient is dispersed homogeneously therein as by stirring. The molten homogeneous mixture is then poured into conveniently sized molds, and allowed to cool and thereby solidify.

Preferably the compound is administered orally.

Preferably, the pharmaceutical preparation is in unit dosage form. In such form, the preparation is subdivided into unit doses containing appropriate quantities of the active component, e.g., an effective amount to achieve the desired purpose. The quantity of active compound in a unit dose of preparation may be varied or adjusted from about 0.1 mg to 1000 mg, more preferably from about 1 mg to 500 mg, according to the particular application.

The actual dosage employed may be varied depending upon the requirements of the patient and the severity of the condition being treated. The determination of the proper dosage for a particular condition is within the skill of the art. Generally, treatment is initiated with smaller dosages which are less than the optimum dose of the compound. Thereafter, the dosage is increased by small amounts until the optimum effect under the circumstances is reached. For convenience, the total daily dosage may be divided and administered in portions during the day if desired.

The amount and frequency of administration of the compounds of the invention and the pharmaceutically acceptable salts thereof will be regulated according to the judgment of the attending clinician considering such factors as age, condition and size of the patient as well as severity of the symptoms being treated. A typical recommended

dosage regimen is oral administration of from 1 mg to 2000 mg/day, preferably 10 to 1000 mg/day, in one to four divided doses to achieve relief of the symptoms. The compound is non-toxic when administered at therapeutic doses.

The following are examples of pharmaceutical dosage forms which contain the compound of the invention. As used therein, the term "active compound" is used to designate one of the compounds of the formula I or salt thereof.

Pharmaceutical Dosage Form Examples

EXAMPLE A

| Tablets | | | |
|---|---|---|---|
| No. | Ingredients | mg/tablet | mg/tablet |
| 1. | Active compound | 100 | 500 |
| 2. | Lactose USP | 122 | 113 |
| 3. | Corn Starch, Food Grade, as a 10% paste in Purified Water | 30 | 40 |
| 4. | Corn Starch, Food Grade | 45 | 40 |
| 5. | Magnesium Stearate | 3 | 7 |
| | Total | 300 | 700 |

Method of Manufacture

Mix Items No. 1 and 2 in a suitable mixer for 10 to 15 minutes. Granulate the mixture with Item No. 3. Mill the damp granules through a coarse screen (e.g., 1/4", 0.63 cm) if necessary. Dry the damp granules. Screen the dried granules if necessary and mix with Item No. 4 and mix for 10-15 minutes. Add Item No. 5 and mix for 1 to 3 minutes. Compress the mixture to appropriate size and weigh on a suitable tablet machine.

EXAMPLE B

| Capsules | | | |
|---|---|---|---|
| No. | Ingredient | mg/capsule | mg/capsule |
| 1. | Active compound | 100 | 500 |
| 2. | Lactose USP | 106 | 123 |
| 3. | Com Starch, Food Grade | 40 | 70 |
| 4. | Magnesium Stearate NF | 4 | 7 |
| | Total | 250 | 700 |

Method of Manufacture

Mix Items No. 1, 2 and 3 in a suitable blender for 10 to 15 minutes. Add Item No. 4 and mix for 1 to 3 minutes. Fill the mixture into suitable two-piece hard gelatin capsules on a suitable encapsulating machine.

## Claims

1. A compound of the formula

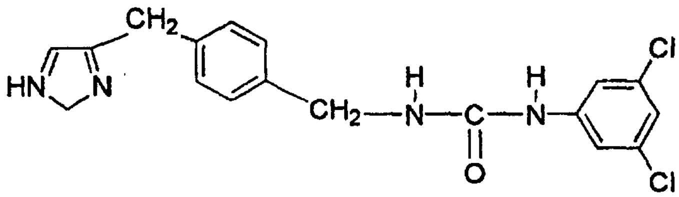

or a salt, tautomer or solvate thereof.

2. A pharmaceutical composition comprising an effective amount of the compound of Claim 1 or a salt, tautomer or solvate thereof in combination with a pharmaceutically acceptable carrier or excipient.

3. The pharmaceutical composition of Claim 2 in combination with a histamine $H_1$ receptor antagonist.

4. The pharmaceutical composition of Claim 3, wherein the histamine $H_1$ receptor antagonist is selected from astemizole, azatadine, azelastine, brompheniramine, cetirizine, chlorpheniramine, clemastine, carebastine, descarboethoxyloratadine, diphenhydramine, doxylamine, ebastine, fexofenadine, loratadine, levocabastine, mizolastine, norastemizole or terfenadine.

5. The pharmaceutical composition of Claim 4 wherein the histamine $H_1$ receptor antagonist is selected from loratadine, descarboethoxyloratadine, fexofenadine and cetirizine.

6. Use of the compound of Claim 1 or a salt, tautomer or solvate thereof in the manufacture of medicament for the treatment of allergy, inflammation, cardiovascular diseases, hypertension, glaucoma, sleeping disorders, diseases of the GI tract, states of hyper and hypo motility of the gastrointestinal tract, disturbances of the central nervous systems, diseases of the central nervous system, hypo and hyperactivity of the central nervous system, Alzheimer's, schizophrenia, obesity and migraine.

7. Use according to Claim 6 for the treatment of inflammation.

8. Use according to Claim 6 for the treatment of allergy.

9. Use according to Claim 6 for the treatment of diseases of the GI-tract.

10. Use according to Claim 6 for the treatment of cardiovascular disease.

11. Use according to Claim 6 for the treatment of disturbances of the central nervous system.

12. Use according to Claim 6 of the compound of Claim 1 or a salt, tautomer or solvate thereof, in combination or admixture with a histamine $H_1$ receptor antagonist in the manufacture of a medicament for the treatment of upper airway allergic responses.

13. Use according to Claim 12 wherein the histamine $H_1$ antagonist is selected from: astemizole, azatadine, azelastine, brompheniramine, cetirizine, chlorpheniramine, clemastine, carebastine, descarboethoxyloratadine, diphenhydramine, doxylamine, ebastine, fexofenadine, loratadine, levocabastine, mizolastine, norastemizole or terfenadine.

14. Use according to Claim 13 wherein the $H_1$ antagonist is selected from: loratadine, descarboethoxyloratadine, fexofenadine, cetirizine.

15. Use according to Claim 14 wherein the $H_1$ antagonist is loratadine

16. Use according to Claim 15 wherein the $H_1$ antagonist is descarboethoxyloratadine.

**Patentansprüche**

1. Verbindung mit der Formel

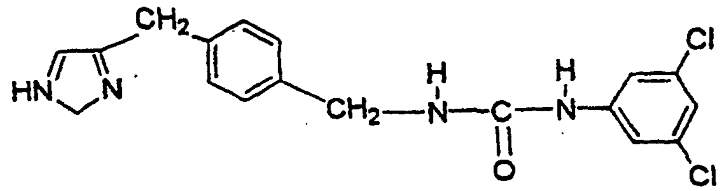

oder ein Salz, Tautomer oder Solvat davon.

**2.** Pharmazeutische Zusammensetzung, die eine wirksame Menge der Verbindung gemäß Anspruch 1 oder ein Salz, Tautomer oder Solvat davon in Kombination mit einem pharmazeutisch annehmbaren Träger oder Hilfsstoff enthält.

**3.** Pharmazeutische Zusammensetzung nach Anspruch 2 in Kombination mit einem Histamin-$H_1$-Rezeptorantagonisten.

**4.** Pharmazeutische Zusammensetzung nach Anspruch 3, bei der der Histamin-$H_1$-Rezeptorantagonist ausgewählt ist aus Astemizol, Azatadin, Azelastin, Brompheniramin, Cetirizin, Chlorpheniramin, Clemastin, Carebastin, Descarboethoxyloratadin, Diphenhydramin, Doxylamin, Ebastin, Fexofenadin, Loratadin, Levocabastin, Mizolastin, Norastemizol oder Terfenadin.

**5.** Pharmazeutische Zusammensetzung nach Anspruch 4, bei der der Histamin-$H_1$-Rezeptorantagonist ausgewählt ist aus Loratadin, Descarboethoxyloratadin, Fexofenadin und Cetirizin.

**6.** Verwendung der Verbindung gemäß Anspruch 1 oder eines Salzes, Tautomers oder Solvats davon zur Herstellung eines Medikaments zur Behandlung von Allergie, Entzündung, Herz-Kreislauf-Erkrankungen, Bluthochdruck, Glaukom, Schlafstörungen, Krankheiten des Gastrointestinaltrakts, Zuständen der Hyper- und Hypomobilität des Gastrointestinaltrakts, Störungen des Zentralnervensystems, Krankheiten des Zentralnervensystems, Hypo- und Hyperaktivität des Zentralnervensystems, Alzheimer-Krankheit, Schizophrenie, Fettleibigkeit und Migräne.

**7.** Verwendung nach Anspruch 6 zur Behandlung von Entzündung.

**8.** Verwendung nach Anspruch 6 zur Behandlung von Allergie.

**9.** Verwendung nach Anspruch 6 zur Behandlung von Krankheiten des Gastrointestinaltrakts.

**10.** Verwendung nach Anspruch 6 zur Behandlung von Herz-Kreislauf-Erkrankung.

**11.** Verwendung nach Anspruch 6 zur Behandlung von Störungen des Zentralnervensystems.

**12.** Verwendung nach Anspruch 6 von der Verbindung gemäß Anspruch 1 oder einem Salz, Tautomer oder Solvat davon in Kombination oder Mischung mit einem Histamin-$H_1$-Rezeptorantagonisten bei der Herstellung eines Medikaments zur Behandlung von allergischen Reaktionen der oberen Luftwege.

**13.** Verwendung nach Anspruch 12, bei der der Histamin-$H_1$-Rezeptorantagonist ausgewählt ist aus Astemizol, Azatadin, Azelastin, Brompheniramin, Cetirizin, Chlorpheniramin, Clemastin, Carebastin, Descarboethoxyloratadin, Diphenhydramin, Doxylamin, Ebastin, Fexofenadin, Loratadin, Levocabastin, Mizolastin, Norastemizol oder Terfenadin.

**14.** Verwendung nach Anspruch 13, bei der der $H_1$-Antagonist ausgewählt ist aus Loratadin, Descarboethoxyloratidin, Fexofenadin, Cetirizin.

**15.** Verwendung nach Anspruch 14, bei der der $H_1$-Antagonist Loratadin ist.

**16.** Verwendung nach Anspruch 15, bei der der $H_1$-Antagonist Descarboethoxyloratadin ist.

**Revendications**

1. Composé de formule

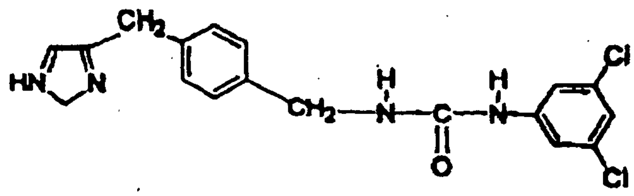

ou un sel, un tautomère ou un solvate qui en dérive.

2. Composition pharmaceutique comprenant une quantité efficace du composé selon la revendication 1 ou un sel, un tautomère ou un solvate qui en dérive en combinaison avec un support ou un excipient pharmaceutiquement acceptable.

3. Composition pharmaceutique selon la revendication 2, en combinaison avec un récepteur antagoniste de l'histamine $H_1$.

4. Composition pharmaceutique selon la revendication 3, dans laquelle le récepteur antagoniste de l'histamine $H_1$ est choisi parmi l'astémizole, l'azatadine, l'azélastine, la bromphéniramine, la cétirizine, la chlorphéniramine, la clémastine, la carébastine, la descarboéthoxyloratadine, la diphénhydramine, la doxylamine, l'ébastine, la féxofénadine, la loratadine, la lévocabastine, la mizolastine, le norastémizole ou la terfénadine.

5. Composition pharmaceutique selon la revendication 4, dans laquelle le récepteur antagoniste de l'histamine $H_1$ est choisi parmi la loratidine, la descarboéthoxyloratadine, la féxofénadine et la cétirizine.

6. Utilisation du composé selon la revendication 1 ou un sel, un tautomère ou un solvate qui en dérive dans la fabrication de médicament pour le traitement de l'allergie, de l'inflammation, des maladies cardiovasculaires, de l'hypertension, du glaucome, des désordres du sommeil, des maladies du tractus gastro-intestinal, des états d'hyper ou d'hypo motilité du tractus gastro-intestinal, des perturbations du système nerveux central, des maladies du système nerveux central, de l'hypo et de l'hyper activité du système nerveux central, de la maladie d'Alzheimer, de la schizophrénie, de l'obésité et de la migraine.

7. Utilisation selon la revendication 6, pour le traitement de l'inflammation.

8. Utilisation selon la revendication 6, pour le traitement de l'allergie.

9. Utilisation selon la revendication 6, pour le traitement des maladies du tractus gastro-intestinal.

10. Utilisation selon la revendication 6, pour le traitement de maladie cardio-vasculaire.

11. Utilisation selon le revendication 6, pour le traitement des perturbations du système nerveux central.

12. Utilisation selon la revendication 6, du composé de la revendication 1 ou un sel, un tautomère ou un solvate qui en dérive, en combinaison ou en mélange avec un récepteur antagoniste de l'histamine $H_1$ dans la fabrication d'un médicament pour le traitement des réponses allergiques des voies aériennes supérieures.

13. Utilisation selon la revendication 12, dans laquelle l'antagoniste de l'histamine $H_1$ est choisi parmi l'astémizole, l'azatadine, l'azélastine, la bromphéniramine, la cétirizine, la chlorphéniramine, la clémastine, la carébastine, la descarboéthoxyloratadine, la diphénhydramine, la doxylamine, l'ébastine, la fofénadine, la loratadine, la lévocabastine, la mizolastine, le norastémizole ou la terfénadine.

14. Utilisation selon la revendication 13, dans laquelle l'antagoniste $H_1$ est choisi parmi : la loratadine, la descarboéthoxyloratadine, la féxofénadine, la cétirizine.

**15.** Utilisation selon la revendication 14, dans laquelle l'antagoniste $H_1$ est la loratadine.

**16.** Utilisation selon la revendication 15, dans laquelle l'antagoniste $H_1$ est la descarboéthoxyloratadine.